# EUROPEAN PATENT APPLICATION

(11) **EP 4 635 300 A1**
(43) Date of publication of application: **22.10.2025**
(21) Application number: 25170931.7
(22) Date of filing: 16.04.2025
(51) Int. Cl.: A23K 10/18, A23K 10/30, A23K 20/10, A23K 20/163, A23K 50/40, A23L 29/238, A23L 29/244, A23L 33/105, A23L 33/135, A23L 33/21, A61K 9/00, A61K 35/57, A61K 35/744, A61K 36/064, A61K 36/28, A61K 36/33, A61K 36/185, A61K 36/9068, A61K 31/733, A61K 39/395

(54) **COMPOSITIONS BASED ON PLANT INGREDIENTS, PROBIOTICS AND IMMUNOGLOBULINS AND THEIR USE IN THE ZOOTECHNICAL FIELD**

(30) Priority: 18.04.2024 IT 202400008863
(71) Applicant: ITALFEED S.r.l. - Società Unipersonale, 20125 Milano (IT)
(72) Inventor: CANDOTTI, Giulia, 20125 Milsn (IT); BIANCHI, Cinzia, 20125 Milano (IT)
(74) Representative: Trupiano, Federica

(57) **Abstract**

Object of the present invention is a composition comprising a mixture that comprises, or alternatively consists of, powdered plant raw materials and/or powdered plant ingredients, dry yeast, soluble fibers and/or prebiotics, probiotics, and egg products. Furthermore, object of the present invention is a composition comprising a mixture comprising, or alternatively consisting of, powdered plant raw materials and/or powdered plant ingredients, dry yeast, soluble fibers and/or prebiotics, probiotics and egg products, said composition being for use in a method to promote intestinal health by supporting the microbiota and for the treatment and/or prevention of disorders of the digestive system, preferably for the treatment and/or prevention of intestinal disorders, in the zootechnical field, particularly in the veterinary field.

## Description

Object of the present invention is a composition comprising a mixture that comprises, or alternatively consists of, powdered plant raw materials and/or powdered plant ingredients, dry yeast, soluble fibers and/or prebiotics, probiotics, and egg products. Furthermore, object of the present invention is a composition comprising a mixture comprising, or alternatively consisting of, powdered plant raw materials and/or powdered plant ingredients, dry yeast, soluble fibers and/or prebiotics, probiotics and egg products, said composition being for use in a method to promote intestinal health by supporting the microbiota and for the treatment and/or prevention of disorders of the digestive system, preferably for the treatment and/or prevention of intestinal disorders, in the zootechnical field, particularly in the veterinary field.

### Technical background

Over the past few decades, there has been a progressive growth in the importance of nutraceuticals in health care, both for human and animal/zootechnical/veterinary use. In particular, this second area of use appears to be in strong growth thanks also to the fact that the concept of health related to pets has changed considerably since the end of the last century, a time when it shifted from the demand related to the simple absence of disease states, to the concept of prevention, to the current demand related to well-being understood as an overall harmonious state of health, physical strength and serenity. For this reason, there's a need to provide functional food/supplements available in the zootechnical/veterinary field as well, which are able to positively influence the health of the consuming animal, in addition to their nutritional value. For example, said functional food/supplements should act by improving metabolic and physiological processes and/or reducing the risks of disease onset.

The digestive system of pets such as dogs and cats, originally carnivorous animals, must be properly nutritionally supported to prevent a wrong diet, for example, predominantly grain-based, from affecting its functionality thus causing disorders of various kinds and more or less severe, such as, for example, diarrhea, constipation, malnutrition determined by poor absorption of food. Furthermore, as is the case with humans, the health of the digestive system in animals is also closely linked to the overall health status and the possible occurrence of allergies, kidney and liver diseases, immune system balance and growth, among others.

Thus, there is still a high need to have novel effective compositions and/or mixtures for the support of the digestive system, particularly the intestinal tract of pets, preferably dogs and cats, as well as for the prevention and treatment of disorders affecting said digestive system, that can be used as dietary supplements. Said compositions and/or mixtures should preferably be of natural origin, have no side effects and be effective in supporting the health of the gastrointestinal tract of the animals for which they are intended. Furthermore, said compositions and/or mixtures should preferably be administered to said animals either by extemporaneous addition to the daily food ration or be able to be added to animal feed at the production stage, thus giving rise to nutritionally enriched functional food.

### Objects of the invention

An object of the present invention is to provide a composition and/or mixture based on powdered raw materials/plant ingredients, dry yeast, soluble fibers/prebiotics, probiotics and egg products for use in zootechnical field, preferably in veterinary field.

Another object of the present invention is the use of the composition and/or mixture according to the present invention as a dietary supplement for the support of the digestive system of animals, as well as for the prevention and treatment of disorders affecting said digestive system, and in particular, the intestinal tract of animals, preferably pets, for example dogs and/or cats.

Further object of the present invention is to provide a functional food for animals, preferably pets, such as, for example, dogs and/or cats, supplemented with said composition and/or mixture.

These and other objects are achieved by the subject matter of the present invention, which relates to a composition and/or mixture based on powdered raw materials/plant ingredients, dry yeast, soluble fibers/prebiotics, probiotics and egg products for use in zootechnical/veterinary field.

### Description of the invention

As a result of intensive research, the Applicant has developed innovative compositions and/or mixtures as well as their uses for the support of the digestive system of animals and the prevention and treatment of disorders affecting said system, particularly the intestinal tract, as detailed in the present description.

Object of the present invention is a composition (briefly, composition of the invention) comprising (I) and, optionally, (II), wherein:
(I) is a mixture (briefly, mixture of the invention) comprising or, alternatively, consisting of:
   (a) at least one powdered raw material/plant ingredient selected from: powdered prickly pear (cladodes, botanical name *Opuntia ficus-indica*), powdered ginger (rhizome, botanical name *Zingiber officinalis*), powdered chamomile (flowers, botanical name *Matricaria chamomilla),* powdered mallow (leaves, botanical name *Malva sylvestris),* and mixtures thereof;
   (b) at least one soluble fiber/prebiotic selected from: inulin, oligosaccharides constituted by mannose and β-glucan units, powdered psyllium cuticle (seeds, botanical name *Plantago ovata)* and mixtures thereof;
   (c) at least one dry yeast;
   (d) at least one probiotic;
   (e) at least one egg product; and
(II) is at least one additive and/or excipient of pharmaceutical or food grade.

Said (a) at least one powdered raw material/plant ingredient selected from: powdered prickly pear (cladodes, botanical name *Opuntia ficus-indica*), powdered ginger (rhizome, botanical name *Zingiber officinalis*), powdered chamomile (flowers, botanical name *Matricaria chamomilla),* powdered mallow (leaves, botanical name *Malva sylvestris)* and mixtures thereof is preferably a mixture of at least two powdered raw materials/ingredients of plant origin selected from those listed. Even more preferably, it is a mixture of at least three of said powdered raw materials/ingredients of plant origin. According to the preferred aspect of the invention, said (a) is a mixture of all four said listed powdered raw materials/ingredients of plant origin.

In the present invention, by raw material/plant ingredient is denoted a raw material/ingredient derived from one or more parts of a plant, as set forth, obtained by any technique known to the person skilled in the art or, according to a preferred aspect of the invention, purchased as a finished product. For example, they may be pulverized plant raw materials and/or dried plant extracts. Said raw materials/plant ingredients will meet all current legislative requirements for use in nutritional, human and/or zootechnical field and may be as such or supported by appropriate carriers, for example, being in a mixture with maltodextrins. According to a preferred aspect of the invention, said raw materials/plant ingredients are derived from plants obtained by organic farming techniques.

Preferably, said powdered prickly pear is a raw material derived from the cladodes of *Opuntia ficus-indica* containing polysaccharides used for their soothing activity, and with a fiber content ≥40%.

Preferably, said powdered ginger is derived from the rhizome of *Zingiber officinalis* and has a high gingerol content. According to the most preferred aspect of the invention, it is a dry extract of the rhizome of *Zingiber officinalis,* with a gingerol content around 5% by weight to the total weight of the powder.

Preferably, said powdered chamomile is derived from the flowers of *Matricaria chamomilla* and has an apigenin content of not less than 0.2%. According to a preferred aspect, said powdered chamomile is a dry powdered extract of the flowers of *Matricaria chamomilla.*

Preferably, said powdered mallow is a raw material derived from the leaves of *Malva sylvestris* belonging to Italian cultivars.

According to a preferred aspect of the present invention, said (a), at least one powdered raw material/plant ingredient selected from: powdered prickly pear (cladodes, botanical name *Opuntia ficus-indica*), powdered ginger (rhizome, botanical name *Zingiber officinalis*), powdered chamomile (flowers, botanical name *Matricaria chamomilla),* powdered mallow (leaves, botanical name *Malva sylvestris)* and mixtures thereof, is present in said mixture (I) in an amount of 8% to 16% by weight to the total weight of the mixture (I), preferably in an amount of 12% to 14% by weight.

According to a preferred aspect of the present invention, said (b) at least one soluble fiber/prebiotic selected from: inulin, oligosaccharides consisting of mannose and β-glucan units, psyllium cuticle powder (seeds, botanical name *Plantago ovata)* and mixtures thereof, is a mixture of at least two of said soluble fibers/prebiotics, even more preferably it is a mixture of inulin, oligosaccharides and psyllium.

Preferably, said inulin is inulin derived from chicory (botanical name *Cichorium intybus*) and consists of oligo- and polysaccharides mainly made of fructose units linked together by β(2-1) bonds in which almost every fructose chain ends in a glucose unit. The number of fructose or glucose units in inulin (degree of polymerization) is preferably between 2 and 60.

Preferably, said oligosaccharides consisting of mannose and β-glucan units are obtained from *Saccharomyces cerevisiae.*

Preferably, said powdered psyllium cuticle is derived from the seeds of *Plantago ovata,* in particular, from the seed cuticle, and has a carbohydrate content, including fiber, around 90%.

According to a preferred aspect of the invention, said (b) at least one soluble fiber selected from: inulin, oligosaccharides consisting of mannose and β-glucan units, psyllium and mixtures thereof, is present in said mixture (I) in an amount of 30% to 60% by weight to the total weight of the mixture (I), preferably an amount of 40% to 50% by weight.

According to a preferred aspect of the invention, said (c) at least one dry yeast, it is brewer's yeast derived from heat-inactivated cells of *Saccharomyces cerevisiae,* preferably with a crude protein content of 38% to 45% by weight to the total weight of the yeast.

Preferably, said (c) at least one dry yeast is present in said mixture (I) in an amount of 30% to 50% by weight to the total weight of the mixture (I), preferably an amount of 40% by weight.

According to a preferred aspect of the invention, said (d) at least one probiotic is of the *Enterococcus faecium* species, even more preferably is the strain of *Enterococcus faecium* (Strain ID: DSM 10663 and NCIMB 10415). Preferably said (d) is the product currently marketed as Oralin^{®} 350G which contains 3.5 x 10¹⁰ CFUs (colony forming units) per gram of *Enterococcus faecium* (Strain ID: DSM 10663 and NCIMB 10415) supported by a lactose carrier.

Preferably, said (d) at least one probiotic is present in said mixture (I) in an amount of 0.5% to 2% by weight to the total weight of the mixture (I), preferably an amount of 0.5% to 1% by weight.

According to a preferred aspect, said (e) at least one egg product is a dried egg product in powdered form which is rich in natural immunoglobulins, with a protein content of 40% to 50% by weight, preferably around 47% by weight, to the total weight of the egg product and a fat content of 38% to 42% by weight, preferably around 40% by weight, to the total weight of the egg product. Preferably, said (e) is the product currently marketed as Bloomy^{®} Boost (Globigen^{®} IC CONC CF) consisting of 98.6% egg powder (additional excipients: silica and dextrose). Preferably, said (e) at least one egg product is present in said mixture (I) in an amount of 0.5% to 2% by weight to the total weight of the mixture (I), preferably an amount of 0.8% to 1.2% by weight.

A preferred mixture (I) according to the present invention is that set forth in qualitative-quantitative terms in Table I below.

**Table I: Qualitative-quantitative composition of the mixture (I) according to a preferred aspect of the present invention.**

| **Component** | | **%** |
|---|---|---|
| (a) | Dry extract of ginger (rhizome, *Zingiber officinalis*) | 1 |
| | Dried extract of chamomile (flowers, *Matricaria chamomilla)* | 2 |
| | Prickly pear powder (cladodes, *Opuntia ficus-indica*) | 2 |
| | Mallow powder (leaves, *Malva sylvestris)* | 5 |
| (b) | Inulin from chicory | 25 |
| | Oligosaccharides (mannose and β-glucan) from *Saccharomyces cerevisiae* | 20 |
| | Psyllium cuticle powder (seeds, *Plantago ovata)* | 3.2 |
| (c) | Brewer's yeast (*Saccharomyces cerevisiae,* inactivated cells) | 40 |
| (d) | Oralin^{®} 350G (*Enterococcus faecium* DSM 10663, NCIMB 10415) | 0.8 |
| (e) | Bloomy^{®} Boost | 1 |

| **Component** | | **%** |
|---|---|---|
| Total | | 100 |

According to an aspect of the present invention, the composition consists of (I), a mixture as shown in Table 1, and (II), at least one additive and/or excipient of pharmaceutical or food grade.

Said (II) at least one additive and/or excipient may be, for example but not limited to, a cereal flour; an anti-caking agent, binder, coagulant, emulsifier, stabilizer, thickener, gelling agent; a sweetener; a flavoring agent; a coloring agent; an antioxidant; a preservative; and other auxiliary substances known to the skilled in the art.

According to a preferred aspect of the present invention, the mixture of the invention and/or the composition of the invention consist of a solid product in powder or granule form which may be dosed and added extemporaneously to the daily food ration of the pet and mixed with it so that it is ingested.

According to another aspect of the invention, said mixture and/or composition can be added to the ingredients that will constitute the pet food during their production process, for example but not limited to a mixture of powdered ingredients, as in the state of the art, then subjected to the treatments/processes that will transform it into dry foods and kibble, wet food, snacks and veterinary supplements according to processes known to the skilled in the art.

According to a further aspect, said mixtures and/or compositions according to the invention may be formulated for oral administration per se, in a liquid form, such as solutions, emulsions, suspensions, sprays; or, more preferably, in a solid form, such as tablets, capsules, granules, powders and equivalent forms known to the skilled in the art. For example, said mixtures and/or compositions are in the form of granules, packaged in single-dose sachets or sticks.

The production methods of these forms are known to the skilled in the art, who can select, for their preparation, any technique suitable for the purpose.

Object of the present invention are the compositions of the invention set forth in the present description, comprising: (I) the mixture of the invention, comprising or consisting of (a), (b), (c), (d), (e) and, optionally, (II) at least one additive and/or excipient of pharmaceutical or food grade, said compositions being for use in a method of preventive and/or symptomatic support and/or treatment of symptoms or disorders of the digestive system, in particular, the intestinal tract, in the zootechnical field, preferably in the veterinary field.

Preferably, the compositions of the invention for use in a method of support and/or treatment and/or prevention of digestive system symptoms or disorders in zootechnical/veterinary field as specified above comprise the mixture (I) as shown in Table I and, optionally, (II) at least one additive and/or excipient of pharmaceutical or food grade.

Said compositions and/or mixtures of the invention for use in a method of support and/or treatment and/or prevention of symptoms or disorders of the digestive system in the zootechnical/veterinary field are administered orally, preferably together with food.

In particular, the composition and/or mixture according to the present invention has been shown to being able to keep the gastrointestinal system in balance and promote intestinal well-being by supporting the microbiota, regularizing stool production and limiting odor. Furthermore, by promoting a healthy intestinal environment, the composition and/or mixture of the invention influences the activity of the immune system positively.

By "irregular stool production" is herein meant overproduction compared to normal production and/or the production of stool of abnormal appearance and consistency. For example, but not limited to, diarrheal episodes with liquid or semi-liquid stools. Preferably, the composition and/or mixture of the invention is meant for use in the treatment of pets, in particular, but not limited to, dogs and cats.

The appropriate dosage of the composition and/or mixture of the present invention will depend, for example, on the type of animal to which it is administered, its size and the health condition of said animal. For example, the mixture (I) described in Table I can be administered in a daily amount from 5 g/kg to 20 g/kg, preferably, according to the present invention, it can be administered in a daily amount from 200 mg to 1000 mg, in a single administration or divided into multiple administrations.

According to another aspect of the invention, when said mixture and/or composition is added to the ingredients that will constitute the food for pets during their production process, it will preferably be present in an amount between 0.5% and 2% to the total weight of the complete feed.

The composition and/or mixture of the invention can be administered as a single treatment and/or in combination with other compositions or therapies (i.e., with adjuvant action) useful in the preventive and/or curative and/or symptomatic treatment of symptoms and/or disorders of the digestive system, as previously described.

By "support and/or treatment method" in the context of the present invention is meant an intervention, comprising the administration of a substance, or mixture of substances or combination thereof, having the purpose of maintaining the state of balance of the treated district of the organism and/or the prevention or reduction/decrease of symptoms or disorders related to a state of imbalance.

Unless otherwise specified, a statement that a composition "comprises" one or more components or substances means that other components or substances may be present in addition to the one, or those, specifically stated. For example, if desired or necessary, other extracts or active substances that contribute to the effectiveness of the composition and/or mixture may also be added to said substances (a), (b), (c), (d) and (e).

Unless otherwise specified, the expression composition comprises a component in a quantity "comprised in a range from x to y" means that said component may be present in the composition in all amounts in said range, even if not made explicit, extremes of the range included.

The mixture and/or composition of the present invention is prepared by mixing the individual constituent components or by any other method deemed valid by the skilled in the art.

Another object of the present invention is a food that comprises in it the mixture and/or composition of the invention.

The composition and/or mixture according to the present invention have several advantages. As documented in the experimental part below, said composition and/or mixture showed to be able to support and improve the health of the digestive system of animals to which it is administered, thanks to a synergistic action exerted by the different components in it. Furthermore, said composition keeps the gastrointestinal system in balance and promotes the intestinal well-being by supporting the microbiota.

Furthermore, said composition and/or mixture has been appropriately designed both to be able to be used as such, dosed and mixed into the daily and usual food of the animal, to which it is intended to be administered, and to be able to be integrated into animal feed by adding it during the production process of said food, thus giving rise to nutritionally enriched functional food, as well as by adding it into veterinary snacks or supplements.

This aspect is a significant advantage because it allows a high degree of flexibility of the product according to the invention, and a consequent reduction in costs that would result from producing separate compositions and/or mixtures depending on their use.

Another object of the present invention is a food containing the composition and/or mixture as previously described.

The present invention will now be shown, in the Experimental section below, in an embodiment thereof with effectiveness data, for illustrative and non-limiting purpose.

### Experimental section

### Example 1- in vitro study

The *in vitro* study, developed by the team at CIAM's research and development laboratory (NIL ITALY, Nutraceutical Interdisciplinary Laboratory, which specializes in in-vitro models for veterinary research) is aimed at evaluating the effect of the composition according to the present invention on the luminal microbiota and intestinal mucosa of the dog, in terms of metabolite production and modulation of bacterial groups.

The composition according to the invention was tested by using the simulator of the gastrointestinal tract and intestinal microbial system (SCIME^{™}), using a long-term colon incubation protocol in the experiment. The SCIME^{™} configuration used for the study also includes the mucosal environment simulator (M-SCIME^{™} configuration), to analyze the bacteria that colonize the intestinal mucosa. The M-SCIME^{™} experimental protocol has a duration of several weeks (as described in the following paragraphs) during which a defined amount of feed (referred to as Feed) is added twice a day, for the entire experiment, to reproduce the undigested portion of feed that is not assimilated in the upper gastrointestinal tract and reaches the colonic environment, to be fermented and metabolized by the intestinal microbiota. The trial according to the present invention lasted for the experimental period of 6 weeks. During the last two weeks, the composition according to the present invention, in particular the composition as shown in Table 1, was administered and added to the Feed. During the trial, samples were collected to perform metabolomic and gene analyses. The analysis of the volatile fatty acid was carried out in parallel during the trial.

The purpose of the trial was to evaluate the effect of the composition according to the present invention on the canine intestinal microbiota under physiological conditions. In particular, the M-SCIME^{™} configuration used (Verstrepen L, Van den Abbeele P, Pignataro G, et al. "Inclusion of small intestinal absorption and simulated mucosal surfaces further improve the Mucosal Simulator of the Canine Intestinal Microbial Ecosystem" (M-SCIMETM). Res Vet Sci; 140. Epub ahead of print 2021. DOI: 10.1016/j.rvsc.2021.08.011) allowed the impact of the ingredient on both luminal and mucosal microbiota bacteria to be analyzed at the same time.

The basic unit of the system consists of a reactor reproducing the upper gastrointestinal tract (St-SI: stomach and small intestine) and two reactors reproducing proximal and distal colon (PC and DC), respectively. During the course of the experiment, the system is fed by solutions that mimic the standard feed (Feed) and pancreatic juice mixture (PJ: Pancreatic Juice).

The study involves 2 donors: in particular, a dog more accustomed to spending time outside and an indoor dog, so as to provide a more comprehensive framework.

In particular, two fecal donors were selected to represent different dog types and habits. "Donor 1" (D1), Lola, is a neutered toy poodle with house habits who does not hangs out with other dogs, whereas "Donor 2" (D2), Lenù, is a neutered springer spaniel used to go out 3 times a day and spend a lot of time outdoors, even with other dogs.

What differentiates PC (proximal colon) and DC (distal colon) is the pH set, since this difference allows the microbiota to better colonize the system as it would in the proximal and distal colons under in vivo conditions. The entire system is maintained at 39°C and under anaerobic conditions with nitrogen gas. The experiment is controlled by software, with a customized program managing all actions (pH control, pump actions, nitrogen flushing, etc.). The two ST-SI vessels receive Feed every 12 hours, the liquid is moved from ST-SI to PC, from PC to DC, and from DC to the waste container by peristaltic pumps. The pH in the colon is constantly monitored and adjusted in the correct range by an acid/base system, following the physiological condition of the dog.

The proposed and developed experimental scheme comprises the following steps: a stabilization period (2 weeks); a control period (2 weeks); and a treatment period (2 weeks) in which the composition according to the invention was administered to the system. During the treatment period, the composition according to the invention was administered twice daily. During these two weeks, the same samplings done during the control weeks were carried out, and the collected data were compared with those from the previous control step to assess statistical differences.

During the trial, samples were collected to assess the following parameters: short-chain fatty acid (SCFA) production, ammonia production and canine microbial composition (the microbial community composition will be determined by Illumina sequencing). The samplings performed were as follows: 3 samples/colon/week for SCFA and ammonia and 2 samples/colon/week for microbial composition (1 for luminal microbiota and 1 for mucosal microbiota).

The sampling of each colonic compartment, PC and DC, of the SCIME was performed 3 times per week during the stabilization, control periods and during the first week of treatment. During the second week of treatment an additional sampling was performed to increase the available data. Liquid samples for subsequent analysis of microbial metabolic activity were immediately frozen at -20 °C, whereas the pelletized cells from the liquid sample were frozen at -20 °C for subsequent molecular analysis. Short-chain C2-C6 fatty acids, including C4-C6 isoforms, were measured by gas chromatography.

After the end of the actual experimental part, sample purification procedures for the sequencing analyses (which give information about the bacterial population of the microbiota and its modulation following the treatment) and analysis for quantification of the ammonium metabolite production were carried out.

The purpose of the trial was to analyze the effect of the composition according to the present invention on the canine microbiota under physiological conditions. In terms of metabolite production, the short-chain fatty acids, in particular acetate, propionate and butyrate, are the primary end products of bacterial fermentation of undigestible dietary fibers. VFAs promote local mucosal health and integrity by stimulating the gastrointestinal epithelial blood flow, promoting intestinal mucosal integrity and serving as a primary energy source for colonocytes.

At a first analysis focusing on VFA (volatile fatty acid) production, a significant increase in Butyrate in DC (distal colon) and a positive trend in Acetate in both DC (distal colon) and PC (proximal colon) were observed.

The results of the tests carried out showed the following positive effects:

### Increase of alpha diversity

The composition according to the invention provides fermentable substrates that can be used by different microbiota bacteria. A significant increase in bacterial diversity was also seen.

### Increase of butyrate production

Butyrate is a major source of energy for colonocytes and high butyrate production is associated with intestinal health. The higher production of butyrate during treatment with the composition according to the present invention is due both to a direct effect (increasing the % of bacteria directly producing butyrate) and an indirect effect (favoring bacteria producing lactate).

### Increase of acetate production

Acetate, the result of reductive acetogenesis, is the net product of fermentation by many of the anaerobic bacteria in the microbiota. Acetate production should be interpreted biologically as beneficial in that it is capable of acidifying the intestinal pH in the micro-environment. It has been highlighted that the composition according to the invention is able to acidify the intestinal pH by increasing the secretion of organic acids, such as for example acetic acid, thus creating a more favorable environment for the physiological microbiota by disfavoring pathogen colonization.

### Increase in the proportion of potentially beneficial bacteria

The treatment with the composition of the present invention shows an increase in bacteria belonging to the phyla Firmicutes (Lactobacillus, Enterococcus, Faecalitalea, Holdemanella, Blautia) and Bacteroidota (Bacteroides).

### Decrease in the proportion of potentially pathogenic bacteria

Especially in the mucosal microbiota, we observe a decrease in bacteria belonging to potentially pathogenic families, such as Enterobacteriaceae and Pseudomonadaceae. In conclusion, the parameters analyzed in the above study are as follows:
- Production of volatile fatty acids (acetate, propionate, butyrate)
- Bacterial composition and diversity (DNA sequencing)
- Ammonium production

The results of the tests carried out showed the following.

The composition according to the present invention stimulated the production of:
✔ Butyrate (+): Source of energy for intestinal cells, promotes colon health and has anti-inflammatory properties
✔ Acetate (+): Acidifies the intestinal environment, creating a favorable ecosystem for beneficial bacteria
✔ Propionate (+): Promotes intestinal balance and lipid metabolism
with a significant increase in butyrate in the distal colon (p-value=0.033) and acetate increasing in the proximal and distal colons (positive trend).

An increase in beneficial bacteria was observed:
✔ Lactobacillus and Enterococcus (improve nutrient assimilation and digestive health)
✔ Firmicutes (support the production of butyrate and propionate)
✔ Bacteroides (improve fiber degradation and VFA metabolism) and a reduction in potentially harmful bacteria:
   ✔ Enterobacteriaceae and Pseudomonadaceae (associated with dysbiosis and intestinal inflammation)

The composition according to the present invention thus leads to an increase of bacterial diversity: a more diverse microbiota is more resilient and stable.

Improved colonization of the intestinal mucosa by probiotics was also observed.

The composition according to the invention caused an increase in ammonium production, which is physiologically expected given the high percentage of crude protein in the composition itself. However, the increase occurred only in the intestinal lumen, whereas the mucosal microbiota reduced the presence of ammonium-producing bacteria. Furthermore, the effect is transient and stabilizes over time.

Indeed, it has been observed that the controlled increase in ammonium is without negative impacts on the beneficial bacterial flora.

The composition according to the present invention has been shown to be effective for the intestinal health of dogs, with a positive impact on bacterial flora, production of beneficial metabolites and stabilization of digestion.

Since the composition according to the present invention promotes intestinal balance, it is ideal for animals with digestive sensitivities.

Herein below a summary table (Table II) of the effects observed by employing the composition according to the present invention is shown.

**Table II**

| **Parameter** | **Observed effect** | **Statistical significance** |
|---|---|---|
| **Butyrate production (DC)** | ↑ significant | p = 0.033 (Wilcoxon's test) |
| **Acetate production (DC)** | ↑ positive trend | p = 0.082 (PC), p = 0.066 (DC) |
| **Propionate (DC)** | ↑ positive trend, not stable in all subjects | Variability among donors |
| **Bacterial diversity (alpha diversity)** | ↑ | Qualitative observation from sequencing |
| **Enterococcus faecium** | ↑ lumen and mucosa colonization | Consistent with administered strain |
| **Ammonium (DC)** | ↑ mild, controlled, consistent with formulation | Physiological interpretation, not harmful |

### Example 2- in vivo preliminary study

A preliminary study involving 9 medium-sized dogs divided into 3 groups of 3 dogs each was carried out, to which the mixture according to the present invention 15 g/kg (group M) were administered, only the probiotic component (d) in the form of the product Oralin^{®} 350G 1 x 10⁹ CFU/animal (group O) or a placebo (group P).

The dogs selected for the preliminary study were of medium size, weighing between 10 kg and 25 kg and mixed breed, and had intestinal disorders for at least 4 days that were manifested by loose, light-colored stools.

The administration of the mixture (I), with composition as shown in Table I below, of the Oralin^{®} 350G product alone or the placebo was done in a single solution, by mixing, for each group, the corresponding product together with the morning food ration and without changing the dog's other eating habits.

**Table I: Composition of the mixture (I) administered to the dogs in group M.**

| **Component** | | **%** |
|---|---|---|
| (a) | Dry extract of ginger (rhizome, *Zingiber officinalis*) | 1 |
| | Dried extract of chamomile (flowers, *Matricaria chamomilla)* | 2 |
| | Prickly pear powder (cladodes, *Opuntia ficus-indica*) | 2 |
| | Mallow powder (leaves, *Malva sylvestris)* | 5 |
| (b) | Inulin from chicory | 25 |
| | Oligosaccharides (mannose and β-glucan) from *Saccharomyces cerevisiae* | 20 |
| | Psyllium cuticle powder (seeds, *Plantago ovata)* | 3.2 |
| (c) | Brewer's yeast *(Saccharomyces cerevisiae,* inactivated cells) | 40 |
| (d) | Oralin^{®} 350G (*Enterococcus faecium* DSM 10663, NCIMB 10415) | 0.8 |
| (e) | Bloomy^{®} Boost | 1 |

| **Component** | | **%** |
|---|---|---|
| Total | | 100 |

A questionnaire was given to dog owners to fill out, containing a subjective assessment of the dog's appearance and number of evacuations, which enabled the results summarized and commented herein below to be recorded. In particular, the owners of the study animals were asked to take note of the stool consistency/appearance with a score scale of 1 to 4 (1=very liquid - 4=normal) and the number of daily evacuations of the dog at time zero (t₀) and for the next 5 days of treatment (t₁₋₅).

In summary, in Table III the data recorded at t₀ and t₅ for the three groups of animals, divided subject by subject (1-9), are set forth.

**Table III:**

| **Group** | **subject (tₓ)** | **consistency** | **n°. of evacuations/day** |
|---|---|---|---|
| **P** | 1 (t₀) | 1 | 5 |
| | 1 (t₁) | 2 | 4 |
| | 2 (t₀) | 2 | 4 |
| | 2 (t₁) | 2 | 4 |
| | 3 (t₀) | 1 | 6 |
| | 3 (t₁) | 2 | 4 |
| **O** | 4 (t₀) | 2 | 4 |
| | 4 (t₁) | 3 | 3 |
| | 5 (t₀) | 2 | 6 |
| | 5 (t₁) | 4 | 3 |
| | 6 (t₀) | 1 | 6 |
| | 6 (t₁) | 3 | 4 |
| **M** | 7 (t₀) | 1 | 6 |
| | 7 (t₁) | 4 | 3 |
| | 8 (t₀) | 2 | 5 |
| | 8 (t₁) | 4 | 2 |
| | 9 (t₀) | 1 | 5 |
| | 9 (t₁) | 4 | 3 |

The preliminary study has shown that the mixture according to the present invention is able to improve, faster and more markedly than probiotic alone, the stool consistency of the dog to which it is administered.

### Example 3 - experimental protocol

Studies related to the assessment of improving the consistency and odor of dog stools by using microbiota analysis are currently underway with veterinary support. The degree of increase in appetite and general improvement in the animal's well-being, including evaluation of weight gain, will also be assessed. The degree of improvement in the animal's skin and coat appearance will then be assessed. Measurements will be made both by subjective tests and thanks to the measurement of specific parameters.

## Claims

1. A composition comprising (I) and optionally (II), wherein:
(I) is a mixture comprising or alternatively consisting of:
(a) at least one powdered raw material/plant ingredient selected from: powdered prickly pear (cladodes, botanical name *Opuntia ficus-indica*), powdered ginger (rhizome, botanical name *Zingiber officinalis*), powdered chamomile (flowers, botanical name *Matricaria chamomilla),* powdered mallow (leaves, botanical name *Malva sylvestris),* and mixtures thereof;
(b) at least one soluble fiber/prebiotic selected from: inulin, oligosaccharides constituted by mannose and β-glucan units, powdered psyllium cuticle (seeds, botanical name *Plantago ovata)* and mixtures thereof;
(c) at least one dry yeast;
(d) at least one probiotic;
(e) at least one egg product; and
(II) is at least one additive and/or excipient of pharmaceutical or food grade.

2. The composition according to claim 1, wherein: said (a) at least one dry extract of plant origin is present in an amount of 8% to 16% by weight to the total weight of the mixture (I); said (b) at least one soluble fiber/prebiotic is present in an amount of 30% to 60% by weight to the total weight of the mixture (I); said (c) at least one dry yeast is present in an amount of 30% to 50% by weight to the total weight of the mixture (I); said (d) at least one probiotic is present in an amount of 0.5% to 2% by weight to the total weight of the mixture (I); and said (e) at least one egg product is present in an amount of 0.5% to 2% by weight to the total weight of the mixture (I).

3. The composition according to claim 1 or 2, wherein said (a) at least one raw material/plant ingredient is a mixture of: powdered prickly pear (cladodes, botanical name *Opuntia ficus-indica*), powdered ginger (rhizome, botanical name *Zingiber officinalis*), powdered chamomile (flowers, botanical name *Matricaria chamomilla*) and powdered mallow (leaves, botanical name *Malva sylvestris).*

4. The composition according to any one of the preceding claims, wherein said (b) at least one soluble fiber/prebiotic is a mixture of inulin, oligosaccharides constituted by mannose and β-glucan units, and powdered psyllium (seeds, botanical name *Plantago ovata).*

5. The composition according to any one of the preceding claims, wherein said (d) at least one probiotic is *Enterococcus faecium,* preferably is the *Enterococcus faecium* Strain ID: DSM 10663 and NCIMB 10415.

6. The composition according to any one of the preceding claims, wherein said (e) at least one egg product is a dried egg product in powdered form which is rich in immunoglobulins, with a protein content of 40% to 50% by weight, preferably around 47% by weight, to the total weight of the egg product, and a fat content of 38% to 42% by weight, preferably around 40% by weight, to the total weight of the egg product.

7. The composition according to any one of the preceding claims, wherein said mixture (I) has the following % composition:
| **Component** | | **%** |
|---|---|---|
| (a) | Dry extract of ginger (rhizome, *Zingiber officinalis*) | 1 |
| | Dried extract of chamomile (flowers, *Matricaria chamomilla)* | 2 |
| | Prickly pear powder (cladodes, *Opuntia ficus-indica*) | 2 |
| | Mallow powder (leaves, *Malva sylvestris)* | 5 |
| (b) | Inulin from chicory | 25 |
| | Oligosaccharides (mannose and β-glucan) from *Saccharomyces cerevisiae* | 20 |
| | Psyllium cuticle powder (seeds, *Plantago ovata)* | 3.2 |
| (c) | Brewer's yeast *(Saccharomyces cerevisiae,* inactivated cells) | 40 |
| (d) | Oralin^{®} 350G (*Enterococcus faecium* DSM 10663, NCIMB 10415) | 0.8 |
| (e) | Bloomy^{®} Boost | 1 |
| Total | | 100 |

8. The composition according to claim 1, for use in a support and/or treatment method, which is preventive and/or symptomatic, of symptoms or disorders of the digestive system, in the zootechnical/veterinary field.

9. The composition for use according to claim 8, wherein said disorders of the digestive system are disorders of the intestinal tract, particularly disorders related to irregular stool production.

10. The composition for use according to claim 8 or 9 in pets, preferably dogs and cats.
